(19) Europäisches Patentamt / European Patent Office / Office européen des brevets

(11) **EP 3 025 758 B1**

(12) **FASCICULE DE BREVET EUROPEEN**

(45) Date de publication et mention
de la délivrance du brevet:
**28.06.2017 Bulletin 2017/26**

(51) Int Cl.:
***A61N 1/37*** *(2006.01)*

(21) Numéro de dépôt: **15196002.8**

(22) Date de dépôt: **24.11.2015**

(54) **DISPOSITIF MÉDICAL IMPLANTABLE ACTIF, NOTAMMENT DE TYPE CAPSULE LEADLESS, AVEC DÉTECTION DE CAPTURE CYCLE-À-CYCLE PAR ANALYSE D'UN SIGNAL D'ACCÉLÉRATION ENDOCARDIAQUE**

AKTIVE IMPLANTIERBARE MEDIZINISCHE VORRICHTUNG, INSBESONDERE VOM TYP LEADLESS-KAPSEL, MIT DETEKTION DER ZYKLUSWEISEN ERFASSUNG DURCH ANALYSE EINES SIGNALS FÜR DIE ENDOKARDIALE BESCHLEUNIGUNG

ACTIVE IMPLANTABLE MEDICAL DEVICE, IN PARTICULAR A LEADLESS CAPSULE, WITH CYCLE-TO-CYCLE CAPTURE DETECTION BY ANALYSING AN ENDOCARDIAL ACCELERATION SIGNAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2014 FR 1461632**

(43) Date de publication de la demande:
**01.06.2016 Bulletin 2016/22**

(73) Titulaire: **Sorin CRM SAS**
**92140 Clamart Cedex (FR)**

(72) Inventeur: **MAKDISSI, Alaa**
**75011 PARIS (FR)**

(74) Mandataire: **Grünecker Patent- und Rechtsanwälte**
**PartG mbB**
**Leopoldstraße 4**
**80802 München (DE)**

(56) Documents cités:
**EP-A1- 2 189 182     EP-A1- 2 412 401**
**WO-A1-2005/089866     US-A1- 2008 021 336**

**Description**

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle concerne notamment, mais de manière non limitative, ceux de ces dispositifs qui se présentent sous forme d'une capsule autonome destinée à être implantée dans une cavité cardiaque, tout particulièrement dans le ventricule. Ces capsules sont dépourvues de toute liaison mécanique et électrique à un dispositif principal distant, implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless*", pour les distinguer des électrodes ou des capteurs disposés à l'extrémité distale d'une sonde (*lead*) conventionnelle, parcourue sur toute sa longueur par un ou plusieurs conducteurs reliant par voie galvanique l'électrode ou le capteur à un générateur connecté à une extrémité opposée, proximale, de la sonde. Une électrode de détection/stimulation en contact avec la paroi du ventricule permet de détecter la présence ou non d'une onde de dépolarisation spontanée de la cavité cardiaque, ainsi que l'instant de survenue de cette onde (marqueur ventriculaire ou auriculaire).

L'électrode permet également de délivrer une impulsion de stimulation en cas de dépolarisation spontanée absente ou tardive, de manière à provoquer la contraction de la cavité cardiaque.

On notera toutefois que le caractère autonome de la capsule n'est pas intrinsèquement une caractéristique nécessaire de la présente invention.

La gestion de l'énergie de stimulation est un aspect critique de tout stimulateur implanté, car il a une incidence directe sur la consommation électrique de la pile intégrée à ce stimulateur, et donc sur sa durée de vie globale.

Ce sujet est particulièrement critique dans le cas d'un stimulateur de type capsule *leadless* où, contrairement aux stimulateurs conventionnels, l'énergie nécessaire à la délivrance de la stimulation constitue 70 % environ de l'énergie totale consommée. De plus, il faut tenir compte de ce que les très faibles dimensions d'une capsule *leadless* imposent de réduire à un minimum la taille de la pile et donc sa capacité, car dans une capsule *leadless* la pile occupe souvent plus de 70 % du volume du dispositif. De fait, si l'on pouvait réduire par exemple de moitié l'énergie nécessaire à la stimulation, on pourrait réduire corrélativement d'environ 40 % la taille de la pile en gardant la même longévité, ce qui permettrait de réduire le volume de la capsule à environ 0,6 cm$^3$ (contre 1 cm$^3$ dans le meilleur des cas actuellement), toutes performances égales par ailleurs.

Pour réduire le plus possible l'énergie dédiée à la stimulation en conservant l'efficacité des impulsions électriques délivrées, on peut employer une technique dite de "capture cycle-à-cycle", qui consiste à maintenir l'énergie de stimulation à un niveau minimal en vérifiant en permanence, après chaque stimulation, si celle-ci a été efficace ("capture") ou non. Si aucune onde de dépolarisation n'a été induite par la stimulation de la cavité cardiaque (absence de capture), l'implant délivre au cours du même cycle cardiaque une stimulation d'une énergie relativement élevée afin de garantir le déclenchement d'une dépolarisation ; ensuite, par itérations successives, l'énergie de stimulation est progressivement réduite à chaque cycle cardiaque, afin de converger à nouveau vers une énergie proche de la limite ou "seuil d'entrainement" nécessaire pour provoquer la dépolarisation de la cavité cardiaque.

Diverses techniques de test de capture ont été proposées. Il est notamment possible d'utiliser un signal délivré par un capteur détectant directement la contraction mécanique du myocarde, ce qui permet d'obtenir immédiatement une information sur la réponse de la cavité cardiaque à la stimulation, en faisant abstraction des périodes de *blanking* et autres limitations inhérentes au recueil d'un signal électrique. En d'autres termes, il s'agit d'utiliser un signal fonctionnel représentatif de la mécanique cardiaque, en lieu et place d'un signal issu de la propagation électrique de l'onde de dépolarisation.

Le EP 2 412 401 A1 (Sorin CRM) décrit un tel dispositif comprenant des moyens de test de capture ventriculaire opérant par analyse d'un signal d'accélération endocardiaque (EA). Le signal EA peut être recueilli notamment au moyen d'une sonde endocavitaire pourvue d'une électrode distale de stimulation implantée au fond du ventricule, intégrant un microaccéléromètre permettant de mesurer l'accélération endocardiaque.

Le test de capture est basé sur l'analyse du signal EA, et notamment de ses composantes successives (composantes EA) correspondant aux bruits majeurs du coeur qu'il est possible de reconnaitre dans chaque cycle cardiaque (sons S1 et S2 du phonocardiogramme). Les variations d'amplitude de la première de ces composantes (composante EA1) sont étroitement liées aux variations de la pression dans le ventricule, tandis que la seconde composante (composante EA2) survient pendant la phase de relaxation ventriculaire isovolumique.

L'analyse peut également prendre en compte la composante secondaire (composante dite EA4 ou EA0) produite par la contraction de l'oreillette, comme dans le cas du EP 2 189 182 A1 (Sorin CRM), qui décrit un dispositif pourvu de moyens d'analyse permettant de reconnaitre dans le signal EA la présence (ou l'absence) d'une composante EA4 afin d'en déduire la présence (ou l'absence) d'une contraction de l'oreillette consécutive à l'application d'une impulsion électrique à cette dernière par les moyens de stimulation auriculaire.

Pour le test de capture ventriculaire décrit par le EP 2 412 401 A1 précité, les composantes EA1 et EA2 du signal EA sont analysées pour en extraire divers paramètres significatifs tels que l'amplitude crête-à-crête des pics PEA1 et PEA2 des composantes EA1 et EA2, l'intervalle temporel entre ces pics PEA1 et PEA2, la largeur à mi-hauteur des composantes EA1 et/ou EA2, les instants de début et de fin de ces composantes, etc. Il peut également s'agir de paramètres morphologiques représentatifs de la forme d'onde du signal EA ou de son enveloppe.

Dans la technique décrite par le EP 2 412 401 A1 précité, ces différents paramètres sont calculés puis regroupés sous forme d'un vecteur représentatif, constitutif d'un point d'un espace vectoriel multidimensionnel.

L'espace vectoriel est ensuite analysé par application d'algorithmes de classification qui permettent de déterminer dans cet espace une frontière entre capture et non-capture. À chaque cycle, un vecteur de paramètres est ainsi constitué à partir du signal EA recueilli, puis sa position dans l'espace vectoriel est évaluée pour décider si la capture est présente ou absente.

Ce type de test de capture ventriculaire est très performant mais sa mise en oeuvre, par des algorithmes complexes d'analyse du signal et de classification dans un espace multidimensionnel, implique des calculs numériques complexes, avec pour corollaire une consommation importante du processeur de l'implant, typiquement de l'ordre de 2 $\mu$W (chiffre qui est à comparer à l'énergie qui est consommée pour la seule délivrance de la stimulation, de l'ordre de 5 $\mu$W).

Si l'on souhaite, notamment pour un stimulateur de type capsule *leadless*, réduire de façon importante l'énergie consommée, le problème consiste à trouver une méthode de vérification de la capture ventriculaire cycle-à-cycle qui, non seulement, réduise l'énergie de stimulation au minimum nécessaire, mais qui, pour autant, n'augmente pas la consommation du circuit électronique, notamment du processeur numérique.

Il est en particulier souhaitable que la consommation de l'électronique associée à la fonction de test de capture cycle-à-cycle ne dépasse pas quelques centaines de nanowatts. De fait, si l'on peut également réduire l'énergie dédiée à la stimulation à une valeur de 1 $\mu$W à 2 $\mu$W (selon le niveau du seuil de stimulation), on arrivera à réduire la taille de la pile de 40 à 60 % par rapport à ce qu'elle est avec les dispositifs actuels.

D'autres techniques de vérification de la capture mettant en oeuvre une détection de la contraction mécanique du coeur sont exposées notamment dans les US 5 549 652 A (test de capture cycle-à-cycle, mais sans exposé détaillé d'une méthode spécifique) et US 6 650 940 B1 (test périodique de capture conventionnel par réduction progressive de l'énergie, sur plusieurs cycles).

L'un des buts de l'invention est de proposer une technique de détection de capture ventriculaire qui minimise le nombre des opérations de calculateur numérique nécessaires et, par voie de conséquence, la consommation électrique du processeur de l'implant.

L'invention propose à cet effet un dispositif comprenant, de manière en elle-même connue :

- un circuit de stimulation ventriculaire, apte à délivrer des impulsions de stimulation de faible énergie à une électrode implantable dans un ventricule d'un patient ;
- un capteur d'accélération, apte à délivrer un signal EA d'accélération endocardiaque ; et
- un circuit de détection de capture ventriculaire, apte à détecter par analyse du signal EA au cours d'un cycle cardiaque la présence ou l'absence d'une contraction du ventricule consécutive à l'application d'une impulsion de stimulation, ce circuit de détection de capture ventriculaire comprenant :

  • des moyens d'échantillonnage du signal EA délivré par le capteur, aptes à délivrer des mesures EA échantillonnées successives ; et
  • des moyens de fenêtrage, aptes à extraire une série desdites mesures EA échantillonnées pendant la durée d'une fenêtre temporelle prédéterminée ouverte après la délivrance d'une impulsion de stimulation.

De façon caractéristique de l'invention, le circuit de détection de capture ventriculaire comprend en outre :

• des moyens sommateurs, aptes à calculer par sommation un indicateur fonction d'une moyenne des valeurs absolues des mesures EA successives de ladite série de mesures EA à la fin de la fenêtre temporelle ; et
• des moyens comparateurs, aptes à comparer la valeur calculée de l'indicateur à un seuil de discrimination prédéterminé, et à décider la présence ou l'absence de capture ventriculaire selon que la valeur de l'indicateur se situe au-dessus ou au-dessous de ce seuil.

Selon diverses caractéristiques subsidiaires avantageuses :

- les moyens sommateurs calculent ledit indicateur indépendamment des valeurs de l'amplitude crête-à-crête des pics du signal EA ;
- la durée de la fenêtre temporelle prédéterminée est comprise entre 75 et 350 ms, et/ou l'instant de début de la fenêtre temporelle prédéterminée est compris entre 5 et 100 ms après la délivrance de l'impulsion de stimulation ;
- le dispositif comprend en outre des moyens de désactivation, aptes à désactiver le circuit de détection de capture ventriculaire après la fin de la fenêtre temporelle et jusqu'au début de la fenêtre temporelle du cycle cardiaque suivant, et pouvant être également aptes à désactiver le circuit de détection de capture ventriculaire entre deux échantillonnages successifs du signal EA délivré par le capteur ;

- les moyens sommateurs sont aptes à calculer ledit indicateur :

· à partir d'un nombre de mesures EA successives qui est un nombre constant d'un cycle cardiaque à l'autre ;
· par sommation des valeurs absolues des mesures EA successives de ladite série de mesures EA ;
· par sommation des valeurs absolues des différences respectives entre : i) les mesures EA successives de ladite série de mesures EA et ii) une valeur qui est une moyenne des mesures EA de ladite série de mesures EA ;
· par sommation des valeurs absolues des différences respectives entre : i) les mesures EA successives de ladite série de mesures EA et ii) une constante de ligne de base ; ou encore
· par sommation des valeurs absolues des différences respectives entre : i) les mesures EA successives de ladite série de mesures EA et ii) la valeur de la première mesure EA de ladite série de mesures EA ;

- le circuit de détection de capture ventriculaire comprend en outre des moyens d'initialisation pour la détermination dudit seuil de discrimination, aptes à : commander le circuit de stimulation ventriculaire pour délivrer une succession d'impulsions de stimulation à énergie maximale ; calculer une moyenne des valeurs des indicateurs respectifs sur ladite succession d'impulsions de stimulation à énergie maximale ; et appliquer un facteur de réduction à ladite moyenne calculée des valeurs des indicateurs, et délivrer le résultat obtenu comme valeur du seuil de discrimination.

On va maintenant décrire un exemple de mise en oeuvre de la présente invention, en référence aux dessins annexés où les mêmes références désignent d'une figure à l'autre des éléments identiques ou fonctionnellement semblables.

La Figure 1 est une vue perspective d'ensemble d'une capsule *leadless.*
La Figure 2 est une vue en coupe longitudinale de la capsule *leadless* de la Figure 1, montrant les principaux composants internes qui la constituent.
La Figure 3 est une série de chronogrammes illustrant, en regard, un signal d'électrogramme EGM, les fenêtres d'analyse pour le test de capture, et le signal d'accélération endocardiaque EA.
Les Figures 4a et 4b sont des chronogrammes montrant plus précisément l'allure du signal EA délivré par un capteur, respectivement un capteur de type capacitif et de type piézoélectrique.

La Figure 5 est une série de chronogrammes expliquant une manière connue d'opérer un test de capture.
La Figure 6 est un organigramme exposant les principales étapes du procédé de détection de capture selon l'invention.
La Figure 7 est une série de chronogrammes explicatifs de l'étape préalable d'initialisation du procédé, permettant de déterminer le seuil de discrimination entre capture et absence de capture.
La Figure 8 est une série de chronogrammes correspondant à un exemple de mise en oeuvre de l'invention, après que le seuil de discrimination a été préalablement déterminé.

On va maintenant décrire un exemple de réalisation du dispositif de l'invention.
En ce qui concerne ses aspects logiciels, l'invention peut être mise en oeuvre par une programmation appropriée du logiciel de commande d'un dispositif stimulateur cardiaque connu, par exemple un stimulateur de type capsule *leadless* endocavitaire.
Ces dispositifs comprennent un microprocesseur programmable couplé à des circuits pour recevoir, mettre en forme et traiter des signaux électriques recueillis par des électrodes implantées, et délivrer des impulsions de stimulation à ces électrodes. L'adaptation de ces dispositifs pour réaliser l'invention est à la portée de l'homme du métier, et elle ne sera pas décrite en détail. En particulier, les logiciels conservés en mémoire et exécutés pourront être adaptés et utilisés pour mettre en oeuvre les fonctions de l'invention qui seront décrites ci-dessous
Le procédé de l'invention est en effet mis en oeuvre par des moyens principalement logiciels, au moyen d'algorithmes appropriés exécutés par un microcontrôleur ou un processeur numérique de signal. Pour la clarté de l'exposé, les divers traitements appliqués seront décomposés et schématisés par un certain nombre de blocs fonctionnels distincts et/ou de circuits interconnectés, mais cette représentation n'a toutefois qu'un caractère illustratif, ces fonctions ou circuits comprenant des éléments communs et correspondant en pratique à une pluralité de fonctions globalement exécutées par un même logiciel.

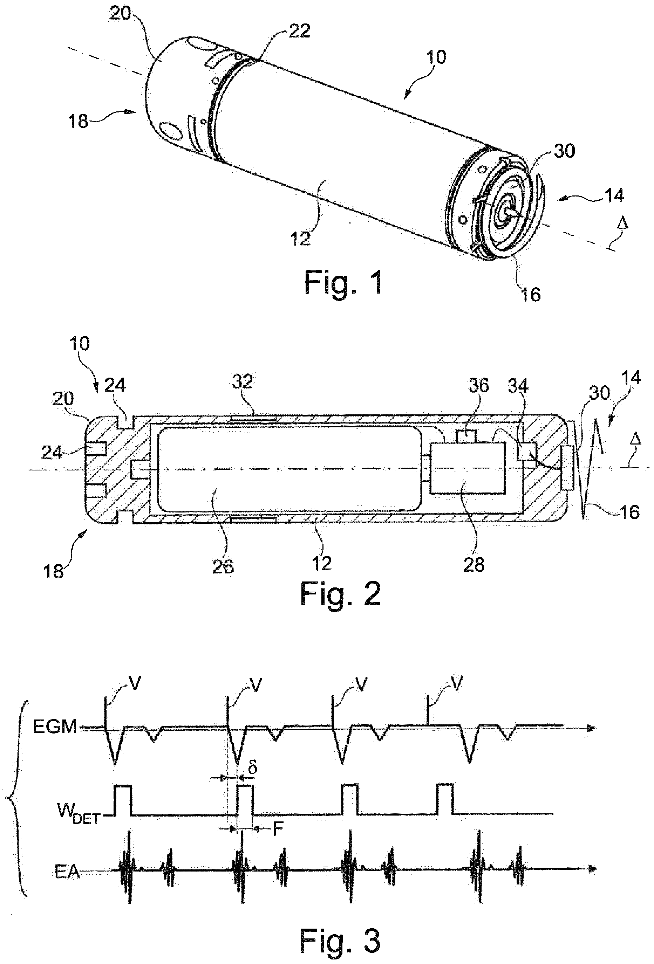

Les Figures 1 et 2 montrent, respectivement en perspective et en coupe longitudinale, un exemple de capsule *leadless,* avec les différents organes qui la constituent.
Sur ces figures, la référence 10 désigne de façon générale la capsule, réalisée sous forme d'un corps tubulaire cylindrique 12 d'axe Δ enfermant les divers circuits électroniques et d'alimentation de la capsule. Les dimensions typiques d'une telle capsule sont un diamètre de l'ordre de 6 mm environ pour une longueur d'environ 25 mm.
À son extrémité distale 14, la capsule porte une vis d'ancrage hélicoïdale 16 permettant sa fixation dans les tissus, par exemple contre une paroi d'une cavité cardiaque. Cette vis peut éventuellement être une vis active, électriquement conductrice, pour le recueil de potentiels de dépolarisation cardiaque et/ou l'application d'impulsions de stimulation. La région proximale 18 de la capsule 10 présente une extrémité arrondie, atraumatique 20 et elle est pourvue de moyens de préhension 22, 24 utilisables pour l'implantation ou l'explantation de la capsule.

Comme illustré Figure 2, la capsule 10 incorpore une pile 26 typiquement avec une densité volumétrique d'énergie de l'ordre de 0,8 à 2 W.h/cm$^3$, un module électronique 28, une électrode frontale 30, et éventuellement une électrode latérale 32. Des traversées telles que 34 permettent de relier les électrodes au module électronique 28.

Le module électronique 28 inclut l'ensemble de l'électronique permettant de piloter les diverses fonctions de l'implant, la mémorisation des signaux recueillis, etc. Il comprend un microcontrôleur et un oscillateur générant les signaux d'horloge nécessaires au fonctionnement du microcontrôleur et à la communication. Il contient également un convertisseur analogique/numérique et une mémoire de stockage numérique. Il peut également contenir des moyens émetteurs/récepteurs pour l'échange d'informations avec d'autres dispositifs implantés par communication de type HBC (*Human Body Communication,* communication par voie intracorpo-relle).

La capsule intègre par ailleurs, de façon caractéristique, un capteur 36 d'accélération endocardiaque (EA) susceptible de délivrer un signal représentatif de l'activité mécanique du myocarde, par exemple un capteur en forme de microaccéléromètre interfacé avec le module électronique 28.

La Figure 3 montre une série de chronogrammes illustrant, en regard, un signal d'électrogramme EGM, des fenêtres d'analyse $W_{DET}$ pour le test de capture, et le signal d'accélération endocardiaque EA.

Après chaque stimulation (marqueur V de dépolarisation stimulée sur l'électrogramme EGM), la mesure du signal EA délivré par l'accéléromètre intégré est activée pendant une fenêtre $W_{DET}$ qui est ouverte soit immédiatement après la délivrance de l'impulsion de stimulation, soit avec un retard δ de l'ordre de 5 à 100 ms. La durée F de la fenêtre $W_{DET}$ est comprise entre 75 et 350 ms. Le contrôle de l'instant de début de la fenêtre de capture $W_{DET}$ et de sa durée est réalisé au moyen du circuit de séquencement du microcontrôleur et du logiciel embarqué qui pilote les circuits électroniques de l'implant.

Le capteur 36 de signal EA peut être un capteur accélérométrique 1 D, 2D ou 3D. De préférence, le capteur est un capteur de type piézoélectrique ou capacitif, mais d'autres types de capteur (optique, résistif, inductif...) capables de générer un signal corrélé au déplacement, à la vitesse ou à l'accélération des parois cardiaques peut être utilisé.

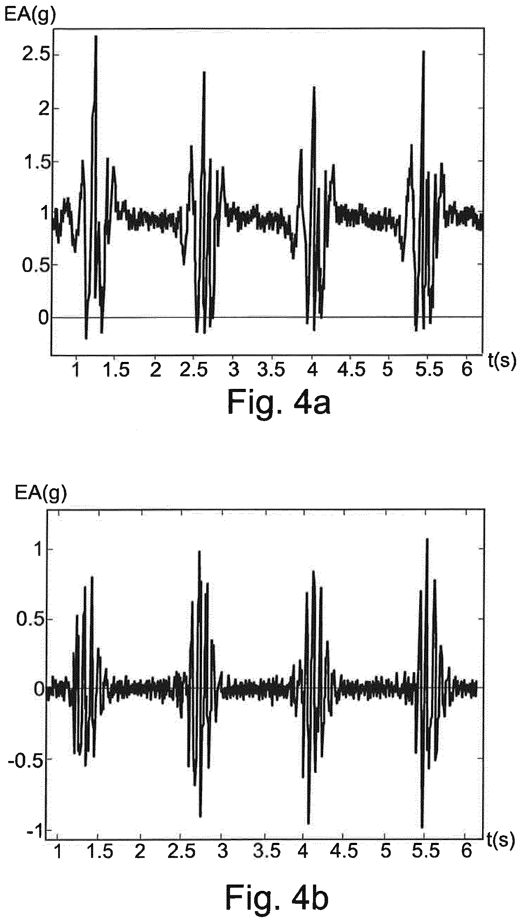

Selon le type de capteur utilisé, le signal EA peut contenir ou non une composante continue. Le signal EA délivré par un capteur de type capacitif MEMS (composant microélectromécanique intégré) présente l'allure générale illustrée Figure 4a, avec une composante continue fonction de l'orientation de la capsule par rapport à la direction de la gravité terrestre. À l'opposé, le signal EA délivré par un accéléromètre de type piézoélectrique fournit un signal tel que celui illustré Figure 4b avec une ligne de base à zéro, car il assure par conception le filtrage de cette composante continue.

Dans l'un ou l'autre cas, le circuit de mesure de test de capture n'est activé que pendant la durée de la fenêtre de mesure d'accélération, le circuit étant totalement ou partiellement éteint (mise en sommeil) le reste du cycle cardiaque. Si la latence du capteur est inférieure à la durée qui sépare deux mesures d'accélération successives, il est même possible d'éteindre le circuit et le capteur entre deux mesures successives du signal EA.

Dans le cas d'un capteur piézoélectrique et de son circuit d'interfaçage, la consommation est de l'ordre de 100 à 200 nW. Si ce capteur n'est activé que pendant la durée de la fenêtre $W_{DET}$ correspondant à 20 à 50 % de la durée du cycle cardiaque, la consommation moyenne d'un tel capteur pourra donc être réduite à une valeur de l'ordre de 50 à 100 nW.

Dans le cas d'un capteur de type capacitif MEMS, la consommation de l'ordre de 300 à 600 nW pourra être réduite de la même façon à une valeur de l'ordre de 150 à 300 nW si le circuit de mesure n'est activé que pendant la durée de la fenêtre de détection $W_{DET}$.

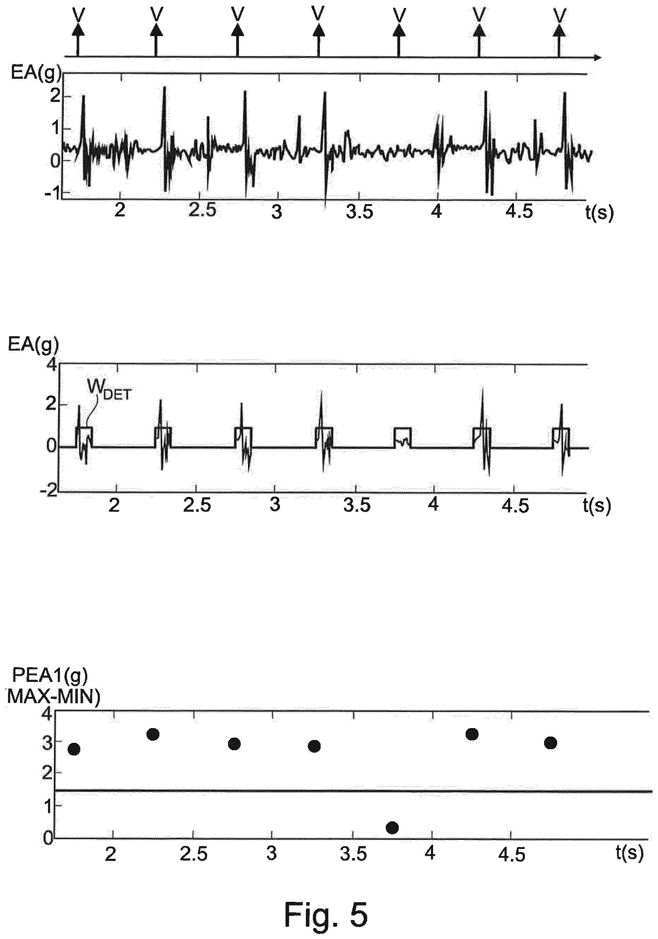

La Figure 5 montre une série de chronogrammes expliquant une manière connue d'opérer le test de capture. On a représenté sur ces chronogrammes, successivement :

- les marqueurs V de dépolarisation stimulée ;
- le signal EA d'accélération endocardiaque ;
- ce même signal, après fenêtrage pendant les périodes de détection $W_{DET}$ ; et
- la valeur calculée du pic d'accélération endocardiaque PEA1, c'est-à-dire la valeur de la différence entre le maximum et le minimum (en valeur algébrique) des valeurs du signal EA échantillonnées au cours de la fenêtre de détection.

Le paramètre de pic d'accélération endocardiaque PEA1 est comparé à un seuil prédéterminé, et l'on voit que par exemple au cinquième cycle cardiaque cette valeur est inférieure au seuil, du fait d'une absence de capture.

Ce signal PEA1, qui n'est basé que sur un écart minimum-maximum, est toutefois très sensible aux bruits de mesure et aux bruits physiologiques engendrés par exemple par la respiration et les mouvements brusques du patient, qui se traduisent par des mouvements correspondants, parasites, du capteur.

C'est pour cette raison que le test de capture n'est généralement pas basé sur l'analyse de ce seul paramètre, mais est combiné à d'autres paramètres représentatifs, comme dans le cas de l'analyse multidimensionnelle décrite par le EP 2 412 401 A1 précité.

L'invention propose, comme cela a été expliqué en introduction, de réaliser un test de capture à partir d'un unique indicateur qui i) requière un minimum de calculs numériques, afin d'économiser l'énergie consommée par le dispositif et ii) soit à lui seul robuste au bruit, de manière à minimiser le risque de fausses détections de capture (faux positifs), susceptibles d'altérer la fiabilité du test.

L'idée de base de la présente invention consiste à utiliser à cet effet un indicateur fonction d'une moyenne des valeurs absolues des mesures du signal EA successives échantillonnées au cours de la fenêtre de détection.

Si l'on désigne par $x_i$, avec i = 1... N, les N mesures d'accélération délivrées par le capteur de la capsule, dans le cas d'un capteur de type piézoélectrique (où le signal EA varie autour d'une ligne de base à zéro), un tel indicateur selon l'invention peut se calculer par :

$$\text{MEAN}_{ABS1} = \frac{1}{N}\sum_{i=1}^{N}|x_1|$$

N peut être typiquement compris entre 20 et 50 selon la fréquence d'échantillonnage.

Sans altérer la pertinence de l'indicateur, on peut éviter l'opération de division par 1/N, qui peut être coûteuse en temps de calcul, si l'on utilise le même nombre N d'échantillons à chaque cycle cardiaque pour le calcul de l'indicateur $\text{MEAN}_{ABS1}$, ce qui donne :

$$\text{MEAN}_{ABS1}' = \sum_{i=1}^{N}|x_1|$$

Le calcul de l'indicateur représentatif se limite ainsi à une simple sommation des N valeurs successives prises par le signal EA au cours de la fenêtre $W_{DET}$ (même si cette fenêtre contient un nombre d'échantillons supérieur à N).

Dans le cas d'un capteur de type capacitif MEMS, la ligne de base du signal d'accélération dépend de l'orientation de la capsule par rapport à la direction de la verticale. La composante d'accélération terrestre (gravité) sera alors présente dans le signal d'accélération indépendamment de l'accélération induite par le mouvement cardiaque et se traduira, comme expliqué plus haut en relation avec la Figure 4a, par une ligne de base non nulle présente dans le signal d'accélération. Cette composante continue doit être supprimée dans le calcul de l'indicateur, qui prend alors la forme :

$$\text{MEAN}_{ABS2} = \frac{1}{N}\sum_{i=1}^{N}|x_1 \quad m|$$

où m représente la valeur moyenne ou la ligne de base du signal d'accélération.

Pour simplifier le calcul, il est possible d'utiliser comme approximation de la valeur de la ligne de base la première valeur $x_1$ mesurée pendant la fenêtre de détection $W_{DET}$ :

$$\text{MEAN}_{ABS3} = \sum_{i=2}^{N}|x_1 - x_1|$$

L'indicateur $\text{MEAN}_{ABS}$ déterminé par l'une des méthodes précédentes est associé à un critère de présence/absence de capture pour son utilisation dans un algorithme de vérification de capture. Le critère utilisé peut notamment consister en une comparaison simple avec un seuil de discrimination déterminé à l'avance.

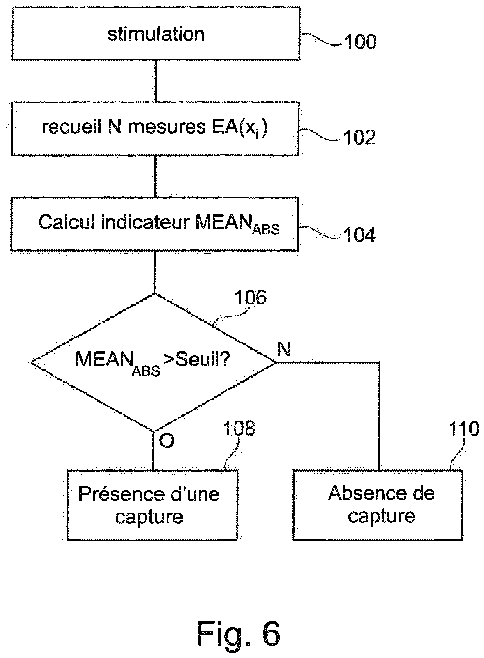

La Figure 6 est un organigramme résumant les différentes étapes du test de capture.

Après une stimulation (étape 100) le dispositif recueille N mesures du signal EA (valeurs $x_i$) successivement échantillonnées à l'intérieur de la fenêtre de détection $W_{DET}$ (étape 102). L'indicateur $\text{MEAN}_{ABS}$ est alors calculé par sommation des valeurs absolues de ces valeurs mesurées $x_i$, ou par sommation des valeurs absolues des différences entre ces valeurs mesurées et une valeur constante reflétant le décalage de la ligne de base par rapport à l'origine (étape 104).

L'indicateur $\text{MEAN}_{ABS}$ calculé est alors comparé à un seuil calculé à l'avance (étape 106). Si l'indicateur est supérieur à ce seuil, alors on considère qu'il y a capture (étape 108) ; dans le cas contraire, on considère qu'il y a absence de capture (étape 110).

Le seuil de discrimination utilisé pour discriminer entre présence et absence d'une capture n'est, de préférence, pas un seuil fixe, mais un seuil calculé automatiquement, pour tenir compte des situations propres à tel ou tel patient et de l'évolution éventuelle de son état clinique sur le long terme.

Ce seuil de discrimination est déterminé au cours d'une phase préalable d'initialisation, de la manière suivante.

Le dispositif déclenche M stimulations successives (typiquement M = 3, 5 ou 10 stimulations) avec des paramètres programmés pour délivrer une énergie maximale, par exemple une tension d'impulsion de 5 à 7 V et une largeur d'impulsion de 1 à 2 ms.

Pour chaque stimulation i, i = 1... M, le dispositif calcule la valeur de l'indicateur $\text{MEAN}_{ABS}$.

Le seuil de stimulation $\text{Cap}_{Threshold}$ est alors déterminé par :

$$\mathbf{Cap_{Threshold}} = \frac{\alpha}{M} \sum_{i=1}^{M} \mathbf{MEAS_{ABS_i}}$$

où $\alpha$ est un facteur de réduction prédéterminé, par exemple $\alpha = 1/2$, 1/3 ou 2/3.

Le résultat de cette détermination est la valeur du seuil de discrimination qui sera appliqué à chaque test de capture ultérieur.

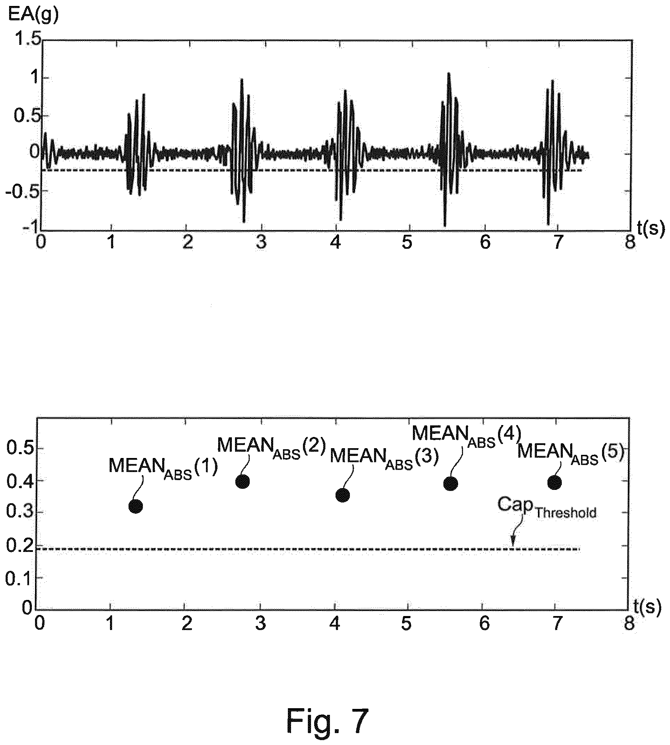

La Figure 7 illustre un exemple de détermination du seuil selon cette technique, avec M = 5 stimulations au cours de la phase d'initialisation.

Le chronogramme du haut montre le signal EA recueilli à la suite de ces stimulations, et le chronogramme du bas les cinq valeurs correspondantes du paramètre $MEAN_{ABS}(i)$. Ces cinq valeurs sont moyennées et un facteur de réduction $\alpha = 1/2$ est appliqué à la moyenne calculée, donnant ainsi une valeur de seuil d'environ 0,185 g (g étant l'accélération de la pesanteur).

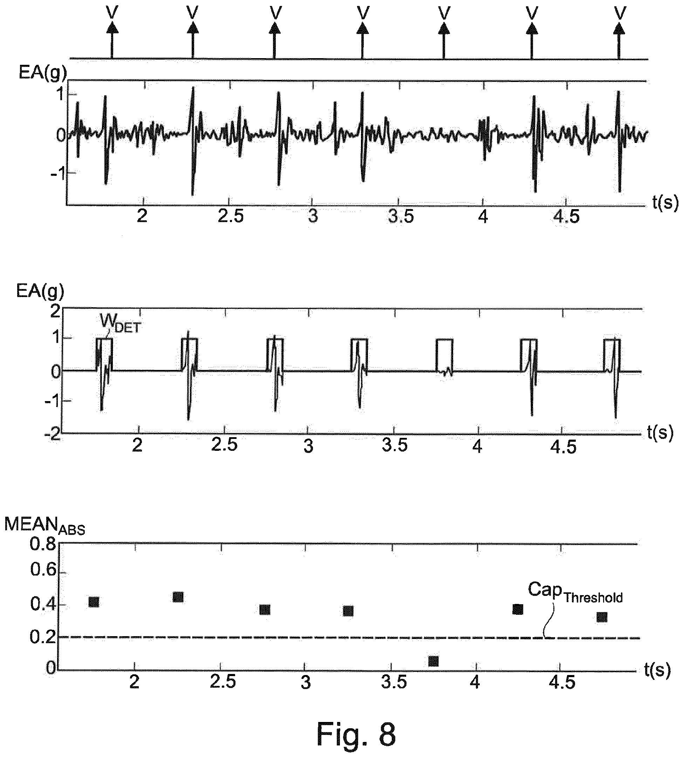

La Figure 8 montre un exemple d'utilisation du signal EA pour un test de capture opéré selon les enseignements de la présente invention. On a représenté sur cette Figure 8 successivement :

- les marqueurs V de dépolarisation stimulée ;
- le signal EA d'accélération endocardiaque ;
- ce même signal, après fenêtrage pendant les périodes de détection $W_{DET}$ ; et
- la valeur calculée du paramètre $MEAN_{ABS}(i)$.

On peut constater que lors de la cinquième stimulation, à t = 3,75 s, le signal d'accélération recueilli pendant la fenêtre de vérification correspondante présente une très faible amplitude. La valeur de l'indicateur $MEAN_{ABS}(5)$ calculée pendant cette fenêtre vaut 0,04 g environ alors qu'il valait 0,4 g environ pour les autres stimulations, qui avaient provoqué une capture. Le seuil de discrimination était dans ce cas fixé à 0,2 g, et on voit qu'il a permis de distinguer très nettement les cycles où la capture est présente de celui où la capture est absente, et ceci avec une excellente immunité aux bruits divers susceptibles de parasiter le signal EA.

On notera que l'indicateur $MEAN_{ABS}$ est très simple à calculer avec un microcontrôleur, car il se résume à une somme de N entiers numériques.

Il est par ailleurs robuste au bruit, car l'opération de sommation revient à opérer un filtrage passe-bas qui réduit très fortement l'incidence des bruits parasites.

Enfin, le critère de test de capture est particulièrement simple à mettre en oeuvre - une simple comparaison entre deux valeurs numériques pour séparer la zone de capture de celle d'absence de capture - donc avec, ici encore, une très grande économie de moyens de calcul.

**Revendications**

1. Un dispositif médical implantable actif (10) du type prothèse cardiaque de stimulation, resynchronisation et/ou défibrillation, comportant :

   - un circuit de stimulation ventriculaire, apte à délivrer des impulsions de stimulation de faible énergie à une électrode implantable dans un ventricule d'un patient ;
   - un capteur d'accélération (36), apte à délivrer un signal EA d'accélération endocardiaque ; et
   - un circuit de détection de capture ventriculaire, apte à détecter par analyse du signal EA au cours d'un cycle cardiaque la présence ou l'absence d'une contraction du ventricule consécutive à l'application d'une impulsion de stimulation,

   le circuit de détection de capture ventriculaire comprenant :

   des moyens d'échantillonnage du signal EA délivré par le capteur, aptes à délivrer des mesures EA échantillonnées successives ; et
   des moyens de fenêtrage, aptes à extraire une série desdites mesures EA échantillonnées pendant la durée d'une fenêtre temporelle ($W_{DET}$) prédéterminée ouverte après la délivrance d'une impulsion de stimulation (V),

   **caractérisé en ce que** le circuit de détection de capture ventriculaire comprend en outre :

   des moyens sommateurs, aptes à calculer par sommation un indicateur ($MEAN_{ABS}$) fonction d'une moyenne des valeurs absolues des mesures EA successives de ladite série de mesures EA à la fin de la fenêtre temporelle ; et
   des moyens comparateurs, aptes à comparer la valeur calculée de l'indicateur à un seuil de discrimination ($Cap_{Threshold}$) prédéterminé, et à décider la présence ou l'absence de capture ventriculaire selon que la valeur de l'indicateur se situe au-dessus ou au-dessous de ce seuil.

2. Le dispositif de la revendication 1, dans lequel les moyens sommateurs comprennent des moyens de calcul dudit indicateur indépendamment des valeurs de l'amplitude crête-à-crête des pics du signal EA.

3. Le dispositif de la revendication 1, dans lequel la durée de la fenêtre temporelle prédéterminée ($W_{DET}$) est comprise entre 75 et 350 ms.

4. Le dispositif de la revendication 1, dans lequel l'instant de début de la fenêtre temporelle prédéterminée ($W_{DET}$) est compris entre 5 et 100 ms après la délivrance de l'impulsion de stimulation (V).

5. Le dispositif de la revendication 1, comprenant en outre des moyens de désactivation, aptes à désactiver le circuit de détection de capture ventriculaire après la fin de la fenêtre temporelle et jusqu'au début de la fenêtre temporelle du cycle cardiaque suivant.

6. Le dispositif de la revendication 5, dans lequel les moyens de désactivation comprennent des moyens de désactivation du circuit de détection de capture ventriculaire entre deux échantillonnages successifs du signal EA délivré par le capteur.

7. Le dispositif de la revendication 1, dans lequel les moyens sommateurs comprennent des moyens de calcul dudit indicateur à partir d'un nombre de mesures EA successives qui est un nombre constant d'un cycle cardiaque à l'autre.

8. Le dispositif de la revendication 1, dans lequel les moyens sommateurs comprennent des moyens de calcul dudit indicateur par sommation des valeurs absolues des mesures EA successives de ladite série de mesures EA.

9. Le dispositif de la revendication 1, dans lequel les moyens sommateurs comprennent des moyens de calcul dudit indicateur par sommation des valeurs absolues des différences respectives entre : i) les mesures EA successives de ladite série de mesures EA et ii) une valeur qui est une moyenne des mesures EA de ladite série de mesures EA.

10. Le dispositif de la revendication 1, dans lequel les moyens sommateurs comprennent des moyens de calcul dudit indicateur par sommation des valeurs absolues des différences respectives entre : i) les mesures EA successives de ladite série de mesures EA et ii) une constante de ligne de base.

11. Le dispositif de la revendication 1, dans lequel les moyens sommateurs comprennent des moyens de calcul dudit indicateur par sommation des valeurs absolues des différences respectives entre : i) les mesures EA successives de ladite série de mesures EA et ii) la valeur de la première mesure EA de ladite série de mesures EA.

12. Le dispositif de la revendication 1, dans lequel le circuit de détection de capture ventriculaire comprend en outre des moyens d'initialisation pour la détermination dudit seuil de discrimination, aptes à :

• commander le circuit de stimulation ventriculaire pour délivrer une succession d'impulsions de stimulation à énergie maximale ;
• calculer une moyenne des valeurs des indicateurs respectifs sur ladite succession d'impulsions de stimulation à énergie maximale ; et
• appliquer un facteur de réduction à ladite moyenne calculée des valeurs des indicateurs, et délivrer le résultat obtenu comme valeur du seuil de discrimination.

**Patentansprüche**

1. Implantierbare aktive medizinische Vorrichtung (10) des Typs Stimulations-, Resynchronisations- und/oder Defibrillations-Herzprothese, umfassend :

- eine Ventrikel-Stimulations-Schaltung, die dazu geeignet ist, Stimulationsimpulse mit geringer Energie an eine implantierbare Elektrode in einem Ventrikel eines Patienten auszugeben;
- einen Beschleunigungssensor (36), der dazu geeignet ist, ein endokardiales Beschleunigungssignal EA auszugeben ; und
- eine Schaltung zur Detektion einer erfolgreichen Stimulation des Ventrikels, die dazu geeignet ist, durch Analyse des EA-Signals während eines Herzzyklus, das Vorhandensein oder die Abwesenheit einer Kontraktion des Ventrikels in Folge des Anlegens eines Stimulationsimpulses zu detektieren,

wobei die Schaltung zur Detektion einer erfolgreichen Stimulation des Ventrikels Folgendes umfasst:

• Mittel zum Abtasten des durch den Sensor ausgegebenen EA-Signals, die dazu geeignet sind, aufeinanderfolgende abgetastete EA-Messungen auszugeben; und
• Mittel zur Fensterung, die dazu geeignet sind, eine Reihe dieser abgetasteten EA-Messungen, während der Dauer eines vorbestimmten Zeitfensters ($W_{DET}$), das nach der Ausgabe eines Stimulationsimpulses (V) anfängt, zu extrahieren,

**dadurch gekennzeichnet, dass** die Schaltung zur Detektion einer erfolgreichen Stimulation des Ventrikels außerdem Folgendes umfasst:

• Summierungsmittel, die dazu geeignet sind, am Ende des Zeitfensters, durch Summierung, einen Indikator ($MEAN_{ABS}$) auszurechnen, der von einem Mittelwert der Absolutwerte der aufeinanderfolgenden EA-Messungen der Reihe von EA-Messungen abhängig ist; und
• Vergleichsmittel, die dazu geeignet sind, den ausgerechneten Wert des Indikators mit einer vorbestimmten Diskriminierungsschwelle ($Cap_{Threshold}$) zu vergleichen, und je nachdem, ob der Wert des Indikators oberhalb oder unterhalb dieses Schwellwerts liegt, das Vorhandensein oder die Abwesenheit einer erfolgreichen Stimulation des Ventrikels zu bestimmen.

2. Vorrichtung nach Anspruch 1, bei der die Summierungsmittel Mittel zur Berechnung des Indikators unabhängig von den Werten der Spitze-zu-Spitze-Amplitude des EA-Signals umfassen.

3. Vorrichtung nach Anspruch 1, bei der die Dauer des vorbestimmten Zeitfensters ($W_{DET}$) zwischen 75 und 350 ms beträgt.

4. Vorrichtung nach Anspruch 1, bei der der Zeitpunkt des Anfangs des vorbestimmten Zeitfensters ($W_{DET}$) zwischen 5 und 100 ms nach der Ausgabe des Stimulationsimpulses (V) liegt.

5. Vorrichtung nach Anspruch 1, weiter umfassend Deaktivierungsmittel, die dazu geeignet sind, die Schaltung zur Detektion einer erfolgreichen Stimulation des Ventrikels nach dem Ende des Zeitfensters und bis zum Anfang des Zeitfensters des darauffolgenden Herzzyklus zu deaktivieren.

6. Vorrichtung nach Anspruch 5, bei der die Deaktivierungsmittel Mittel zur Deaktivierung der Schaltung zur Detektion einer erfolgreichen Stimulation des Ventrikels zwischen zwei aufeinanderfolgenden Abtastungen des durch den Sensor ausgegebenen EA-Signals umfassen.

7. Vorrichtung nach Anspruch 1, bei der die Summierungsmittel Mittel zur Berechnung des Indikators aus einer von einem Herzzyklus zum Nächsten konstant bleibenden Anzahl von aufeinanderfolgenden EA-Messungen umfassen.

8. Vorrichtung nach Anspruch 1, bei der die Summierungsmittel Mittel zur Berechnung des Indikators durch Summierung der Absolutwerte der aufeinanderfolgenden EA-Messungen der Reihe von EA-Messungen umfassen.

9. Vorrichtung nach Anspruch 1, bei der die Summierungsmittel Mittel zur Berechnung des Indikators durch Summierung der Absolutwerte der jeweiligen Unterschiede zwischen i) den aufeinanderfolgenden EA-Messungen der Reihe von EA-Messungen und ii) einem Mittelwert der EA-Messungen der Reihe von EA-Messungen umfassen.

10. Vorrichtung nach Anspruch 1, bei der die Summierungsmittel Mittel zur Berechnung des Indikators durch Summierung der Absolutwerte der jeweiligen Unterschiede zwischen i) den aufeinanderfolgenden EA-Messungen der Reihe von EA-Messungen und ii) einer Basislinienkonstante umfassen.

11. Vorrichtung nach Anspruch 1, bei der die Summierungsmittel Mittel zur Berechnung des Indikators durch Summierung der Absolutwerte der jeweiligen Unterschiede zwischen i) den aufeinanderfolgenden EA-Messungen der Reihe von EA-Messungen und ii) dem Wert der ersten EA-Messung der Reihe von EA-Messungen umfassen.

12. Vorrichtung nach Anspruch 1, bei der die Schaltung zur Detektion einer erfolgreichen Stimulation des Ventrikels außerdem Initialisierungsmittel zur Bestimmung der Diskriminierungsschwelle umfasst, die dazu geeignet sind:

   • die Ventrikel-Stimulations-Schaltung zur Ausgabe einer Reihe von Stimulationsimpulsen mit maximaler Energie anzusteuern;
   • einen Mittelwert der Werte der jeweiligen Indikatoren für die Reihe von Stimulationsimpulsen mit maximaler Energie zu berechnen; und
   • einen Reduktionsfaktor auf den berechneten Mittelwert der Werte der Indikatoren anzuwenden und das erreichte Ergebnis als Wert der Diskriminierungsschwelle auszugeben.

**Claims**

1. An active implantable medical device (10) of the cardiac prosthesis type for stimulation, resynchronization and/or defibrillation, comprising:

   - a ventricular stimulation circuit adapted to deliver low energy stimulation pulses to an implantable electrode in a ventricle of a patient;
   - an acceleration sensor (36), adapted to deliver an endocardial acceleration EA signal; and
   - a ventricular capture detection circuit adapted to detect a presence or absence of a contraction of the ventricle after an application of a stimulation pulse, by analyzing the EA signal during a cardiac signal,

   wherein the ventricular capture detection circuit comprises:

   • means for sampling the EA signal delivered by the sensor, adapted to deliver successive sampled EA measurements ; and
   • windowing means adapted to extract a series of said EA measurements sampled during a duration of a predetermined temporal window ($W_{DET}$) which opens after the application of a stimulation pulse (V),

   **characterized in that** the ventricular capture detection circuit further comprises

> • summing means, adapted to calculate, by summing, an indicator ($MEAN_{ABS}$) based on an average of absolute values of successive EA measurements of said series of EA measurements when the predetermined temporal window ends; and
> • comparator means, adapted to compare the calculated value of the indicator to a predetermined discrimination threshold ($Cap_{Threshold}$); and determine a presence or absence of ventricular capture depending on whether the indicator value is above or below this threshold.

2. The device according to claim 1, wherein the summing means comprise means for calculating said indicator independently of peak-to-peak amplitude values of the EA signal.

3. The device according to claim 1, wherein the duration of the predetermined temporal window ($W_{DET}$) is between 75 and 350 ms.

4. The device according to claim 1, wherein the predetermined temporal window ($W_{DET}$) begins between 5 and 100 ms after delivery of the stimulation pulse (V).

5. The device according to claim 1, further comprising disabling means, adapted to disable the ventricular capture detection circuit after the temporal window ends and until the temporal window begins for the next cardiac cycle.

6. The device according to claim 5, wherein the disabling means comprise means for disabling the ventricular capture detection circuit between two successive samplings of the EA signal delivered by the sensor.

7. The device according to claim 1, wherein the summing means comprise means for calculating said indicator from a number of successive EA measurements, which is a constant number from one cardiac cycle to another.

8. The device according to claim 1, wherein the summing means comprise means for calculating said indicator by summing absolute values of the successive EA measurements of said series of EA measurements.

9. The device according to claim 1, wherein the summing means comprise means for calculating said indicator by summing absolute values of respective differences between: i) the successive EA measurements of said series of EA measurements and ii) a value which is an average of the EA measurements of said series of EA measurements.

10. The device according to claim 1, wherein the summing means comprise means for calculating said indicator by summing absolute values of respective differences between: i) the successive EA measurements of said series of EA measurements and ii) a constant base line.

11. The device according to claim 1, wherein the summing means comprise means for calculating said indicator by summing absolute values of respective differences between: i) the successive EA measurements of said series of EA measurements and ii) the value of the first EA measurement of said series of EA measurements.

12. Device according to claim 1, wherein the ventricular capture detection circuit further comprises initialization means for determining said discrimination threshold, adapted to:

> • control the ventricular stimulation circuit to deliver a series of stimulation pulses with maximum energy;
> • calculate an average of the indicator values of said series of stimulation pulses with the maximum energy; and
> • apply a reduction factor to said calculated average of the indicator values and issue the obtained result as a value for the discrimination threshold.

Fig. 1

Fig. 2

Fig. 3

Fig. 4a

Fig. 4b

Fig. 5

```
                    ┌─────────────────────────┐
                    │      stimulation        │────100
                    └─────────────────────────┘
                                │
                    ┌─────────────────────────┐
                    │  recueil N mesures EA(x_i) │────102
                    └─────────────────────────┘
                                │
                    ┌─────────────────────────┐
                    │ Calcul indicateur MEAN_ABS │────104
                    └─────────────────────────┘
                                │
                              106
                          ╱─────────╲
                         ╱           ╲        N
                        ╱ MEAN_ABS>Seuil? ╲──────────┐
                         ╲             ╱             │
                          ╲─────────╱               │
                              O  108                110
                    ┌─────────────────┐    ┌─────────────────┐
                    │  Présence d'une │    │  Absence de     │
                    │    capture      │    │   capture       │
                    └─────────────────┘    └─────────────────┘
```

# Fig. 6

14

Fig. 7

Fig. 8

**EP 3 025 758 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2412401 A1
- EP 2189182 A1
- US 5549652 A
- US 6650940 B1